# EUROPEAN PATENT APPLICATION

(11) **EP 3 450 012 A1**
(43) Date of publication of application: **06.03.2019**
(21) Application number: 17730226.2
(22) Date of filing: 26.04.2017
(51) Int. Cl.: B01J 20/22, B01J 20/28, B01J 20/30

(54) **METAL-ORGANIC GELS AND METAL-ORGANIC AEROGELS FORMED FROM NANOFIBRES OF COORDINATION POLYMERS**

(30) Priority: 27.04.2016 ES 201630538
(71) Applicant: Universidad del Pais Vasco, 48940 Leioa Vizcaya (ES)
(72) Inventor: VALLEJO SÁNCHEZ, Daniel, E-48940 Leioa - Vizcaya (ES); BEOBIDE PACHECO, Garikoitz, E-48940 Leioa - Vizcaya (ES); CASTILLO GARCÍA, Oscar, E-48940 Leioa - Vizcaya (ES); PÉREZ YÁÑEZ, Sonia, E-48940 Leioa - Vizcaya (ES); LANCHAS GONZÁLEZ, Mónica, E-48940 Leioa - Vizcaya (ES); LUQUE ARREBOLA, Antonio, E-48940 Leioa - Vizcaya (ES); ROMÁN POLO, Pascual, E-48940 Leioa - Vizcaya (ES)
(74) Representative: ABG Intellectual Property Law, S.L.
(86) International application number: PCT/ES2017/070255
(87) International publication number: WO 2017/186997

(57) **Abstract**

The present invention relates to metal-organic gels and metal-organic aerogels made of dithiooxamidate (DTO) or rubeanate ligand-based coordination polymers, method for preparing thereof and use in chemical species capture, separation and/or catalysis, environmental cleanup, metal recovery, passive sampling, among others.

## Description

### Field of the Invention

The present invention relates to the field of metal-organic gels (MOGs) and metal-organic aerogels (MOAs) of coordination polymer, method for preparing thereof and their use in the capture of chemical species and/or separation, detection, catalysis, environmental cleanup, metal recovery, passive sampling, among others.

### Background of the Invention

Porous coordination polymers, also referred to as MOFs (metal-organic frameworks), are characterized by having unique properties as well as multiple functionalities, therefore they have been the focus of countless papers in recent years in the area of physical chemistry, materials science or similar research areas [Zhou, H.C. Chem. Soc. Rev., 2014, 43, 5415-5418] .

Despite the existence of commercial activity involving materials of this type, MOF applications are restricted to technologies that are still in the demonstration phase (gaseous fuel storage in vehicles, catalysis, gas treatment, etc.). This is due to a series of limitations that coordination polymers still have to date, such as: limited pore size, material shaping in a post-synthesis step and a high production cost.

Pore size (2-10 nm) depends on the organic ligand length, where increasing said size not only entails a challenge in terms of synthesis, but also may weaken crystalline structure stability or give rise to interpenetrated structures. The small pore size of the MOFs is particularly interesting for the storage and separation of small molecules that weakly interact with one another, where the narrowness of the gaps strengthens adsorbate-adsorbent interactions. However, in most cases reduced pore sizes entail drawbacks such as prolonged diffusion times or limited accessibility, particularly in applications that are carried out with large molecules, such as non-polymeric macromolecule or biomolecule separation, catalysis or detection, for example [Xuan, W. et al. Chem. Soc. Rev., 2012, 41, 1677-1695] .

As regards material shaping, MOFs are generally produced in powdery form and the processing thereof as a monolith or tablet involves the use of additives which worsen the porous properties [Bazer-Bachi, D. et al., Power Technology, 2014, 255, 52-29] . Although there is extensive literature on MOF synthesis, the shaping thereof has not been widely studied, this being one of the points limiting its application at the industrial level.

On the other hand, despite the existence of MOFs that are produced at competitive costs, many of them still have a high cost today, particularly when it comes to MOFs that have high porosity and pore size values.

One proposal for increasing pore size is based on the preparation of metal-organic gels, also known as metallogels. In this aspect, a gel can be defined as a non-fluid polymeric or colloidal network which expands over the entire volume occupied by a liquid. The solid network is characterized by having a low density and a large pore volume which is occupied by the liquid or solvent in the wet state. Porosity is due to the microstructure, i.e., the cross-linking or aggregation of the particles forming same. Metal-organic gels can be defined as a type of gel in which the solid matrix is formed by metal ion and organic ligand coordination complexes.

The metal-organic gel can be used in wet form (with solvent) or in dry form (aerogel, xerogel or cryogel). By subjecting the gel to a drying process (air, lyophilization, supercritical drying, etc.), solvent molecules are removed thereby creating empty pores. This method allows producing very porous structures, with pore sizes greater than 20 nm and also having a high specific surface area, as well as a markedly low density (generally less than 0.5 g/cm³).

Furthermore, the possibility of producing them directly in monolithic form without requiring subsequent processing is another noteworthy difference of metal-organic gels and metal-organic aerogels. All this contributes to overcoming the drawbacks mentioned for MOFs.

Some examples of MOFs which are processed as gels and/or aerogels prepared with different transition metal ions (Fe³⁺, Ni²⁺, Cu²⁺, Cr³⁺) and ligands such as trimesate (benzene-1,3,5-tricarboxylate), oxalate, 4-aminopyridine or acetylacetonate, have been described in the prior art. Lohe, M.R. et al. (Chem. Commun., 2009, 6056-6058) describe an Fe(III) and benzene-1,3,5-tricarboxylate aerogel (MIL-100(Fe) aerogel) that is proposed as a new route for the application of MOFs as catalysts or catalytic supports due to being microporous/mesoporous or macroporous. Xiang, S. et al (J. Mat. Chem., 2012, 22, 1862-1867) refer to Cr³⁺/Fe³⁺- and carboxylic ligand-based metal-organic aerogels. These compounds are made from carboxylic ligands for producing structures inspired, once again, in the known MIL compounds. Yang, Q. et al. (Micropor. Mesopor. Mater., 2014, 187, 108-113) show the formation process dependency of the Al³⁺/Cr³⁺ and acetylacetonate aerogel with various synthesis parameters.

Aerogels have also been prepared from non-porous coordination polymers (Angulo-Ibañez, A. et al., Polymers, 2016, 8, 16), where a one-dimensional polymer of general formula [M(oxalate) (4-aminopyridine)₂]ₙ in a mesoporous/macroporous monolithic form (pores > 20 nm) is processed.

However, the examples listed in the literature lack any stability comparable to that of conventional porous materials such as zeolites, mesoporous silica or activated carbon. This is because the coordination polymers used in metal-organic gels are based on readily reversible coordination bonds.

Another prior art example is a Cu(II)-oxalate coordination complex-based metal-organic gel (Saha, S., et. al., Chem. Eur. J. 2013, 19, 9562-9568) the polymer of which adopts the form of nanometric fibers having a size comprised between 20 and 45 nm. However, given the acid-base equilibria of the ligand and the strength of the metal-oxalate bond, this metal-organic gel has reduced acid/base stability.

Other examples in the prior art describe coordination polymers based on different transition metals and the DTO ligand (DTO: dithiooxamidate, also known as rubeanate and by its IUPAC name, ethanedithioamidate), but in no case are a gel (be it metallogel, xerogel, aerogel, cryogel...) or nanometric fibers produced from same. In Japanese and United States patents of reference JP5074458 and US2013/0306488, respectively, the Cu-DTO coordination complex is produced in the form of micrometric particles which, once isolated, are used in cathode manufacturing for electrochemical purposes. In the method used, the reaction is performed by mixing rubeanic acid and copper sulfate in an aqueous-ethanolic solution, without adding a base, which gives rise to the production of micrometer-sized particles. Patent GB1006120 discloses information about an electrolyte containing a polymer formed by the DTO ligand and a transition metal which can be Fe(II), Cu(II), Ni(II) or Co(II). Once again, no reference is made to the produced product being similar to a gel, or to it being made up of nanometric fibers. According to the paper by Kitagawa H. et al., published in Synthetic Metals, 119 (2001) 485-486, a copper coordination polymer-based formulation with a DTO derivative forms a two-dimensional structure but not in the form of nanometric fibers.

### Brief Description of the Invention

The authors of the present invention have developed a gel based on a metal-organic matrix formed by a framework of coordination polymer nanometric fibers, where said metal-organic matrix has a high pore volume and a high specific surface area. Furthermore, unlike other metal-organic gels, this material is characterized by withstanding well reducing environments and by being stable in a wide pH range (1-14) and in organic and aqueous solvents.

The metal-organic gel of the present invention is one that can be easily produced, chemically and thermally stable and insoluble in most solvents. The key factor for producing these resistant materials with improved properties is the use of the dithiooxamidate ligand which forms particularly strong coordination bonds with metal ions that are soft or have an intermediate hardness and confers a high chemical stability to the material.

On the other hand, controlling metal-organic gel synthesis allows the growth of the coordination polymer in the form of cross-linked nanometric fibers, giving rise to a three-dimensional structure characterized by having, as mentioned above, high pore volumes and a high specific surface area. Furthermore, said synthesis process leads to the formation of gels enclosing a large amount of solvent and allows producing the material with the shape of the container in which it has been prepared without requiring subsequent processing, so the shape of the end product can be controlled. This, along with their high porosity, confers unique properties to said gels as alternative candidate to porous coordination polymers or MOFs.

Therefore, a first aspect of the present invention relates to a metal-organic gel comprising a metal-organic matrix of cross-linked nanometric fibers, wherein said nanometric fibers comprise coordination polymer chains of general formula (M-DTO)ₙ, where M is a transition metal or a mixture of at least two transition metals; DTO is dithiooxamidate; and n is the number of M-DTO repeating units forming the coordination polymer, n being a number greater than or equal to 10.

In a second aspect, the present invention relates to a method (hereinafter method of the invention) for preparing a metal-organic gel, wherein said method comprises:
a) dissolving or dispersing a transition metal salt or a mixture thereof, in an organic solvent or a mixture of at least two organic solvents;
b) dissolving dithiooxamide and a base in an organic solvent;
c) mixing the solution or dispersion produced in step a) with the solution produced in step b); and
d) allowing the resulting mixture to stand until formation of the metal-organic gel.

An additional aspect of the present invention consists of a metal-organic gel obtainable by the method of the preceding inventive aspect.

In a particular embodiment, the method of the invention further comprises, after step d), a drying step for drying at room pressure and temperature. This process leads to solvent removal at a speed such that it allows microstructural reorganization of the polymer network, thus giving rise to a xerogel.

Accordingly, an additional aspect relates to a xerogel obtainable by the method of the preceding paragraph.

In another particular embodiment, the method of the invention further comprises, after step d), a supercritical drying step for drying in the presence of a supercritical fluid. This step involves exchanging the synthesis solvent with a solvent which is soluble in the supercritical fluid and then removing same.

During this process, the liquid that is inside the gel is extracted, which leads to the formation of an aerogel which maintains the three-dimensional structure of cross-linked nanometric fibers of the metal-organic gel.

Accordingly, an additional aspect relates to an aerogel obtainable by the method of the preceding paragraph.

The metal-organic gel and aerogel, produced as described above, are characterized by having a large pore size and volume along with a high accessible surface area. Specifically, the large pore size makes them excellent candidates for applications such as chemical species capture and separation or catalysis, given that the species of interest can more easily access the active zones of the polymer, unlike what is observed in the so-called MOFs where the small pores generally complicate diffusion processes.

Furthermore, in any of the forms thereof (gel or aerogel), the thiol groups which the metal-organic matrix contains on its surface cause the matrix to be highly selective to soft metals such as Hg and Ag, this capture furthermore being irreversible. This feature is particularly interesting because it makes them viable as passive samplers when determining mercury or its derivatives (methyl-mercury) in rivers, lakes, etc.

Therefore, a final aspect of the invention relates to the use of the gel and aerogel as defined in the preceding paragraph, in chemical species capture, separation and/or catalytic processes, contaminated water cleanup or metal recovery processes and in analytical passive sampling applications.

### Brief Description of the Drawings

Figure 1 shows a photograph of the metal-organic gel.

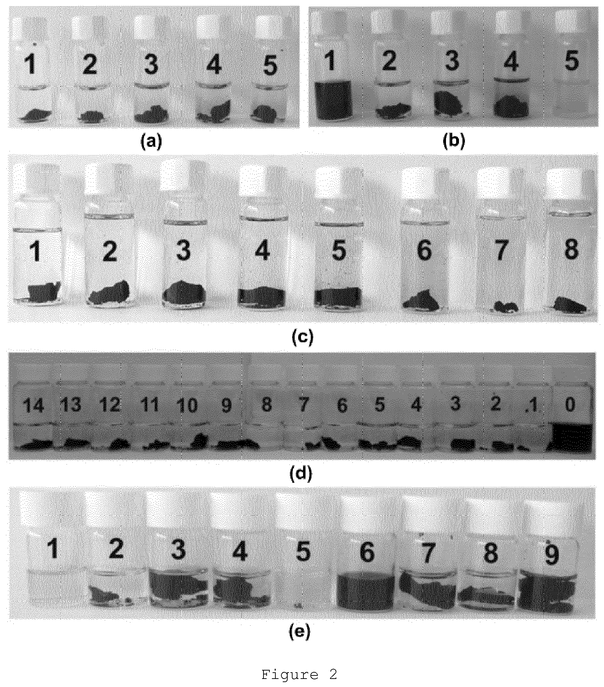

Figure 2 shows the stability of the metal-organic gels in different conditions. a) Polydentate carboxylic acids (1 M) : 1. Terephthalic acid; 2. 2,3-pyrazinedicarboxylate acid; 3. Trimesic acid; 4. Isophthalic acid; 5. Oxalic acid. b) Amines (1 M): 1. Pentylamine; 2. Diethanoldiamine; 3. Pyridine; 4. N,N,N',N'-tetramethylethylenediamine; 5. Diethylenetriamine. c) Conventional solvents: 1. Dichloromethane; 2. DMSO; 3. Toluene; 4. Acetonitrile; 5. Chloroform; 6. Diethyl ether; 7. Pentane; 8. Acetone. d) pH: from 14 to 1, referring to the pH value adjusted with NaOH or H₂SO₄. The vial numbered with 0 is a concentrated 96% H₂SO₄ solution. e) Others: 1. 69% HNO₃; 2. 37% HCl; 3. pure CH₃COOH; 4. 25% NH₃; 5. 30% H₂O₂; 6. 0.2 M I₂; 7. 2.8 M NaBH₄; 8. 1 M KNO₃; 9. 1 M NaCl.
Figure 3 shows the transmission electron microscopy image (TEM) of a metal-organic gel fragment.
Figure 4 shows optical images (left) and transmission electron microscopy (TEM) images (right) for the metal-organic gel (a), metal-organic xerogel (b) and metal-organic aerogel (c) .
Figure 5 shows TEM images of several metal-dithiooxamidate gels: a) CoDTO, b) FeDTO, c) PdDTO, d) RhDTO, e) NiPdDTO and f) NiCoDTO.
Figure 6 shows an image showing the metal capture (in percentage) in metal-organic gels by means of immersing the gel in an aqueous solution with different metal concentrations for 24 hours.

### Detailed Description of the Invention

The first aspect of the invention consists of a metal-organic gel comprising a metal-organic matrix of cross-linked nanometric fibers, wherein said nanometric fibers comprise coordination polymer chains of general formula (M-DTO)ₙ, where M is a transition metal or a mixture of at least two transition metals; DTO is dithiooxamidate; and n is the number of M-DTO repeating units forming the coordination polymer, n being a number equal to or greater than 10.

Throughout this specification and in the attached claims, the term "metal-organic gel" must be understood as a structure comprising a metal-organic matrix dispersed in an organic liquid. In the context of the present invention, the metal-organic matrix corresponds with a structure formed by a framework of cross-linked nanometric fibers, wherein said nanometric fibers comprise a one-dimensional coordination polymer based on repeating units of a transition metal and an organic ligand bound by coordinate covalent bonds.

The term "nanometric fibers" must be understood as structures consisting of continuous polymeric filaments the diameter of which is equal to or less than 100 nm.

In a preferred embodiment, the nanofibers have a diameter comprised between 2 and 300 nm, preferably between 2 and 200 nm, more preferably between 2 and 100 nm, even more preferably between 2 and 50 nm, more preferably between 5 and 40 nm, and even more preferably between 5 and 20 nm.

In another preferred embodiment, the nanofibers have a length comprised between 0.1 and 30 µm, preferably between 0.1 and 20 µm, more preferably between 0.1 and 10 µm, even more preferably between 0.1 and 8 µm, more preferably between 0.1 and 5 µm, even more preferably between 0.2 and 2 µm.

In the present invention, the organic ligand of the coordination polymer, i.e., dithiooxamidate, is the conjugate base of dithiooxamide (H₂DTO; CAS: 79-40-3), a compound which is also known as rubeanic acid or ethanedithioamide and has the chemical formula C₂H₄N₂S₂:

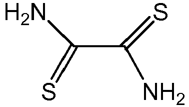

Said organic ligand is coordinated in a bis-bidentate manner to two transition metals, forming a µ-κ²N, S:κ²N',S' bridge. The molecular structure of the coordination polymer can therefore be described according to the following formula (I): 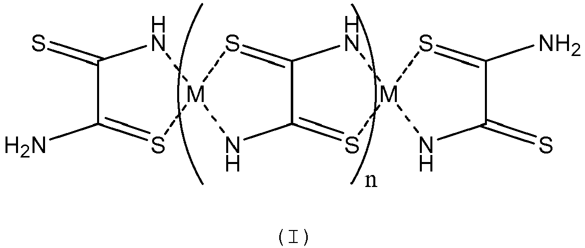 where M is the transition metal or a mixture of at least two transition metals and n are the M-DTO repeating units forming the coordination polymer.

In a particular embodiment, the transition metal is selected from Cr, Rh, Ru, Mn, Fe, Co, Ni, Cu, Zn, Pd, Ag, Au, Cd, Pt and mixtures thereof. Preferably, the transition metal is selected from Ni, Cu, Co, Fe, Rh, Pt, Ru, Pd and mixtures thereof, more preferably Pd, Ni and Cu.

Said transition metals are in the coordination polymer in the form of metal ions, such as Cr³⁺, Mn³⁺, Mn²⁺, Fe²⁺, Co³⁺, Co²⁺, Ni²⁺, Cu²⁺, Cu⁺, Zn²⁺, Pd²⁺, Ag⁺, Au⁺, Cd²⁺, Pt²⁺, Rh²⁺, Ru²⁺, Ru³⁺.

In a particular embodiment, n is a number such that the molecular weight of the coordination polymer is between 1,769 and 235,245 kDa, more preferably between 29,361 and 117,976 kDa.

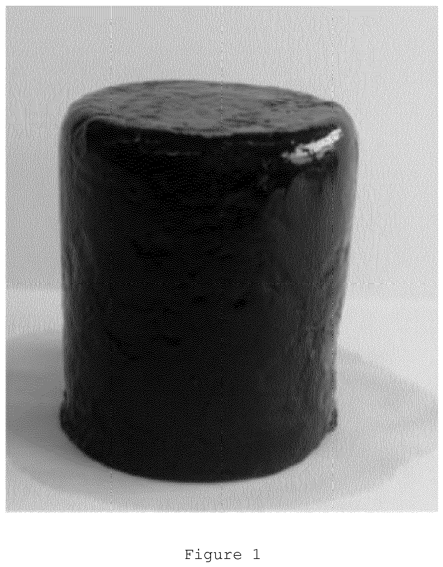

Figure 1 shows a photograph of a metal-organic gel representative of the present invention.

The metal-organic gel of the present invention is characterized by having improved chemical stability, particularly against acidic pHs, organic solvents and reducing conditions, due to the strength of the coordination bond the DTO ligand establishes with the transition metals. Figure 2 shows the stability of the metal-organic gels in different conditions such as in coordinating environments, organic solvents, pH, weak oxidizing environments, reducing environments and saline solutions.

Furthermore, compared to MOFs, they have the advantages mentioned for metal-organic gels given that the metal-organic gel of the invention is obtained with large pore volumes and sizes (> 15 nm), can be prepared in monolithic form without a post-synthesis processing, and is synthesized using a simple and commercially accessible ligand, the dithiooxamide.

Therefore, in a particular embodiment the metal-organic matrix has a pore volume comprised between 0.5 and 10 cm³/g, preferably between 1 and 10 cm³/g. In another particular embodiment, the metal-organic matrix has an average pore size comprised between 2 and 300 nm, preferably between 2 and 100 nm, more preferably between 2 and 50 nm, even more preferably between 2 and 30 nm.

In another particular embodiment, the metal-organic matrix making up the metal-organic gel has a high specific surface area, more particularly said specific surface area is comprised between 100 and 800 m²/g.

"Specific surface area" must be understood as the surface area of the metal-organic matrix divided by its mass (expressed in m²/g) .

In a particular embodiment, the metal-organic gel comprises between 60 and 99% by weight of solvent in which the metal-organic matrix comprising the network of cross-linked nanometric fibers is dispersed.

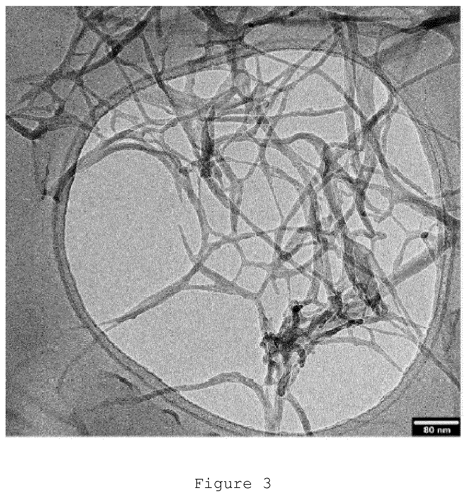

Said solvent fills the pores formed during the assembly of the metal-organic matrix made by the framework of cross-linked nanofibers, as can be seen in Figure 3.

In a second aspect, the invention relates to a method for preparing a metal-organic gel. Said method comprises:
a) dissolving or dispersing a transition metal salt or a mixture thereof, in an organic solvent or a mixture of at least two organic solvents;
b) dissolving dithiooxamide and a base in an organic solvent;
c) mixing the solution or dispersion produced in step a) with the solution produced in step b); and
d) allowing the resulting mixture to stand until formation of the metal-organic gel.

A solution or dispersion of a transition metal salt or a mixture thereof, in an organic solvent or a mixture of at least two organic solvents, is prepared in step a) of the method of the invention.

In a particular embodiment, the transition metal salt is selected from nitrate, chloride, perchlorate, bromide, sulfate, acetate and other organic carboxylates, preferably acetate and other organic carboxylates, more preferably acetate. The degree of hydration (water molecules) of the salt should not be relevant, provided that the purity of the reagent is taken into account when preparing the solution.

In another particular embodiment, the salt of the metal used is based on transition metals selected from Cr, Mn, Fe, Co, Ni, Cu, Zn, Pd, Rh, Ru, Ag, Au, Cd, Pt and mixtures thereof, more preferably Ni, Cu, Pd, Co, Fe, Pt, Rh, Ru or a mixture thereof, and particularly Pd, Ni and Cu.

The transition metal salt solution or dispersion is prepared in an organic medium, without adding water. The organic solvent is selected based on the best fiber formation and gelling conditions. In a particular embodiment, the organic solvent is selected from N,N-dimethylformamide (DMF); N,N-diethylformamide (DEF); dimethylsulfoxide (DMSO); N,N-dimethylacetamide (DMA); alcohols such as methanol, ethanol, iso- or n-propanol, butanol; tetrahydrofuran and a mixture thereof.

The amount of organic solvent will depend on the desired concentration. Therefore, in another particular embodiment the concentration of transition metal salt in the organic solvent is comprised between 0.5 and 500 mM, preferably between 25 and 100 mM. Nevertheless, optimal concentrations differ depending on the metal. Therefore, 85 mM are more preferably used for Pd-DTO, 25 mM for Cu-DTO, 75 mM for Ni-DTO and 100 mM for heterometallic compounds such as NiPd-DTO, NiCu-DTO and PdCu-DTO, for example. In another preferred embodiment, 50 mM are used for heterometallic compounds such as NiPd-DTO, NiCu-DTO and PdCu-DTO, for example.

In a preferred embodiment, the transition metal salt is a Ni, Cu or Pd acetate and the solvent is DMF. In another preferred embodiment, the transition metal salt is a Ni, Cu or Pd acetate and the solvent is DMSO.

In step b) of the method of the invention, the dithiooxamide ligand (H₂DTO) is added to an organic solution further comprising a base.

In a particular embodiment, the base is selected from sodium hydroxide, potassium hydroxide, sodium methoxide, ammonia and alkylamines such as diethylamine and triethylamine, more preferably alkylamines such as diethylamine and triethylamine and even more preferably triethylamine is used. The base is used to promote ligand deprotonation.

In another particular embodiment, the solvent is selected from N,N-dimethylformamide (DMF); N,N-diethylformamide (DEF); dimethylsulfoxide (DMSO); N,N-dimethylacetamide (DMA); alcohols such as methanol, ethanol, iso- or n-propanol, butanol; tetrahydrofuran and a mixture thereof. Preferably, the organic solvent is DMF. More preferably, the organic solvent is DMSO.

Even more preferably, the organic solvent used in step b) is the same as the organic solvent used in step a) of the process of the invention.

Given that the nanometric fibers in the end product comprise repeating units with a stoichiometric ratio of 1:1 (metal-ligand), the ligand concentration must be the same as that obtained in step a) for the metal solution. Therefore, in a particular embodiment the ligand concentration in the organic solution is comprised between 0.5 and 500 mM, preferably between 25 and 100 mM, and even more preferably 85 mM for Pd-DTO, 25 mM for Cu-DTO, 75 mM for Ni-DTO and 100 mM for heterometallic nuclear compounds such as NiPd-DTO, NiCu-DTO and PdCu-DTO, for example. In another preferred embodiment, the ligand concentration in the organic solution is 50 mM for heterometallic compounds such as NiPd-DTO, NiCu-DTO and PdCu-DTO, for example.

If the metal salt used is not soluble in the organic solvent, a dispersion will be obtained instead of a solution. Nevertheless, this detail is not significant for obtaining the desired product.

In step c), the two solutions a) and b) are mixed, or the dispersion a) plus the solution b) are mixed if the transition metal salt is not soluble in the organic solvent.

In a preferred embodiment, the solution containing the DTO ligand is added continuously and in a single step to the transition metal solution or dispersion.

Preferably, the resulting mixture is subjected to stirring or sonication. If stirring is used, a preferable embodiment is to stir the mixture between 100 and 2000 rpm, more preferably at 800 rpm. In the case of mixing by sonication, a preferable embodiment is to use a frequency comprised between 5 and 40 kHz, more preferably 16 kHz. The mixing of a) and b) is preferably done in a temperature range comprised between 0 and 50°C, more preferably between 10 and 30°C.

In step d) of the method of the invention, the produced mixture is allowed to stand until formation of the metal-organic gel. Depending on the starting reagents and on the reaction and mixing conditions, the metal-organic gel can be formed almost immediately, for example, within less than one minute after the mixing process of step c), or it may require more time. In a particular embodiment, the mixture is allowed to stand between 1 and 48 hours, more preferably for 24 hours.

The synthesis process of the invention leads to the formation of a metal-organic gel enclosing a large amount of solvent, particularly between 60 and 99% by weight with respect to the total weight of the metal-organic gel. Furthermore, it allows the metal-organic gel to acquire the shape of the container in which it has been prepared, so the shape of the end product can be controlled, designing it for example, in monolithic form, and therefore without requiring a post-synthesis shaping process.

The metal-organic gel thus produced is characterized by withstanding reducing conditions well and by being stable in a wide pH range (1-14) and in organic and aqueous solvents. Furthermore, it has a high porosity.

Accordingly, an additional aspect of the present invention consists of a metal-organic gel that can be produced according to the process of the invention described above.

In a preferred embodiment, the metal-organic gel produced according to the process of the invention is subjected to a first step of washing with organic solvents leading to the removal of the remaining reagents and synthesis solvents. Preferably, a solvent in which the reagents and the by-products are soluble, more preferably, the synthesis solvent is used.

In a second step, the metal-organic gel free of unreacted species is washed. For this step, the use of solvents that are soluble in CO₂ is preferred, for example alcohols, such as methanol, ethanol or propanol, more preferably ethanol.

Additionally, the organic solvent initially incorporated in the polymeric matrix can be exchanged with another solvent without requiring said solvent to be an organic solvent. In a particular embodiment, said exchange solvent can be water.

In another preferred embodiment, the metal-organic gel produced according to the method of the invention is subjected to a drying process for the purpose of producing a metal-organic xerogel. Said drying method can be performed at room temperature and pressure conditions. In this case, drying can be performed at ambient temperature at a pressure of about 1 bar or, to speed up the process, at higher temperatures (50-150°C) which are provided by an external source, such as an oven, for example.

Preferably, the temperature ranges between 100 and 120°C, whereas the pressure is usually about 1 bar.

Alternatively, or in a combined manner, said drying method can be performed by applying a vacuum.

Even more preferably, this drying method is performed on a metal-organic gel containing a volatile organic solvent such as an alcohol, for example, as the organic solvent. Said solvent can be the one that was used for preparing precursor solutions leading to the production of the metal-organic gel or the one that is used in a subsequent washing step such as the one described above.

This drying process, at ambient pressure and temperatures or by means of providing external heat and/or by applying a vacuum, allows removing the organic solvent, or where appropriate the exchange solvent, and leads to the production of a xerogel. By means of this process, the solvent present in the metal-organic gel is removed at a rate such that it allows reorganizing the microstructure formed by the nanofibers, causing them to collapse and stack to give rise to a framework that virtually lacks porosity but is chemically identical to the metal-organic gel and aerogel.

In another preferred embodiment, the metallogel produced according to the method of the invention is subjected to a supercritical drying process.

Supercritical drying must be understood as a process of removing the solvent contained in the metal-organic gel above its critical point, or by means of a process of exchanging said solvent with a supercritical fluid followed by evaporating said fluid at a temperature greater than its critical temperature.

If the solvent contained in the metal-organic gel is not soluble in the supercritical fluid, said solvent is firstly exchanged with a solvent soluble in said supercritical fluid.

After exchanging the initial solvent (the synthesis solvent of the gel), the new solvent is exchanged with a fluid in subcritical conditions. The temperature of the gel immersed in the fluid (all the gel being inside an airtight container equipped with valves required for correct operation) is then raised until exceeding the critical point of the chosen fluid. Finally, the pressure is gradually reduced to its ambient value in isothermal conditions.

In a preferred embodiment, the supercritical fluid is CO₂. In this case, supercritical drying is performed by first immersing the metal-organic gel in liquid CO₂ at a temperature comprised between 15 and 30°C and a pressure between 35 and 60 bar. The resulting material is then dried in supercritical conditions, i.e., increasing the temperature above the critical temperature of CO₂ (above 31°C) and a pressure between 60 and 150 bar. Finally, the pressure is reduced in isothermal conditions until reaching ambient pressure.

If the solvent contained in the metal-organic gel is not soluble in CO₂, said solvent is first exchanged with a solvent soluble in CO₂, such as an alcohol or an alkane, for example. Said solvent is exchanged afterward by CO₂ in conditions close to 20°C and 50 bar. Supercritical drying is then performed in the same conditions described above, i.e., if CO₂ is used as a supercritical fluid, the system is brought to a temperature above the critical temperature of CO₂ (31°C) and to a pressure between 60 and 150 bar, and the pressure is then reduced in isothermal conditions until reaching ambient pressure.

This supercritical drying process allows removing the solvent contained in the metal-organic gel, giving rise to the production of a metal-organic aerogel.

Furthermore, it has been observed that supercritical drying of the metal-organic gel allows said gel to retain its initial shape, i.e., to keep the three-dimensional structure of the metal-organic matrix formed by the network of cross-linked nanometric fibers, therefore resulting in a highly porous material.

Therefore, an additional aspect of the present invention consists of an aerogel that can be produced according to the method described above.

In the context of the present invention, the term "metal-organic aerogel" must therefore be understood as a porous network of a structure that is identical or similar to that of the metal-organic gel from which it originated, but without the solvent.

In a preferred embodiment, the aerogel is characterized by having pore volumes comprised between 0.5 and 10 cm³/g, preferably between 1 and 10 cm³/g. This allows producing a material with a high specific surface area, particularly the specific surface area of the aerogel can have values ranging between 100 and 800 m²/g. The surface area values can be calculated by means of the BET model [Brunauer, S. et. al. J. Am. Chem. Soc. 1938, 60, 309].

As a result of this drying, a material with very low apparent densities, preferably between 0.5 and 0.01 g/cm³, with respect to its actual density (between 2.0 and 3.5 g/cm³) is produced.

By means of N₂ adsorption isotherm measurements at 77 K, it is found that aerogels have a significant meso- and macroporous contribution, although the average pore size is comprised between 2 and 300 nm, preferably between 2 and 100 nm, more preferably between 2 and 50 nm, even more preferably between 2 and 30 nm.

In a particular embodiment, the nanometric fibers comprised in the aerogel have a diameter equal to or less than 100 nm, preferably comprised between 2 and 100 nm, preferably between 2 and 50 nm, more preferably between 5 and 40 nm, and even more preferably between 5 and 20 nm.

In another particular embodiment, the nanometric fibers comprised in the aerogel have a length comprised between 0.1 and 10 µm, preferably between 0.1 and 8 µm, more preferably between 0.1 and 5 µm, even more preferably between 0.2 and 2 µm.

Due to the chemical stability of the gels and aerogels of the invention, derived fundamentally from the strength of the bonds between the transition metal and the DTO ligand, and their large pore size and high specific surface area, gels and aerogels are excellent candidates for applications in chemical species capture, separation and/or catalytic processes, contaminated water cleanup or metal recovery processes and in analytical passive sampling applications.

The application thereof for the selective capture of soft metals is also particularly relevant. The thiol groups which the aerogel contains on its surface cause the aerogel to be highly selective to soft metals such as Hg and Ag, this capture furthermore being irreversible. This feature is particularly interesting because it makes them viable as passive samplers when determining mercury or its derivatives (methyl-mercury) in rivers, lakes, etc.

Accordingly, the aerogel of the present invention can be used in processes requiring easy permeability of chemical species due to the high specific surface area and mesoporous character. In this context, as particular embodiments of the present invention, the aerogel that can be produced according to the method described above can be used in chemical species capture, separation and/or chemical catalysis, contaminated water cleanup or metal recovery processes and in analytical applications such as passive sampling.

Throughout this specification and in the attached claims, "chemical species capture" must be understood as the irreversible immobilization of chemical species which may include, and are not limited to, free molecules in solution or in gaseous phase, solvates and/or salts, cationic or anionic species and chemical elements. In this context, "contaminated water cleanup processes" must be understood as those processes which allows capturing chemical species in water that belong to the chemical contaminant group, i.e., any organic/inorganic substance in an amount which causes an irritating, corrosive, suffocating or toxic effect in animals or plants. Likewise, "metal recovery" must be understood as the capture of chemical species that belong to the metal and metalloid group.

In this specification and in the attached claims, the "passive sampling" technique must be understood as the collection of chemical species controlled by a physical process such as diffusion, or permeability through the metal-organic framework.

### Example 1. Preparation of a Ni-DTO metal-organic gel

0.933 g of Ni(OAc)₂ were dissolved in 48 mL of DMF/DMA (60:40 vol:vol) with the help of a sonic tip at 80% its power for 2 minutes. The ligand solution was prepared by dissolving 0.451 g of dithiooxamide in 2 mL of DMF/DMA (60:40 vol:vol) together with 523 µL of triethylamine. The dithiooxamidate ligand solution was added at once to the metal dispersion. This addition process was performed in an ultrasonic bath (ULTRASONS-H, Selecta) at a temperature of 15°C until a change in the viscosity of the samples was visually observed (5 minutes). Once the metal-organic gel acquired the suitable consistency, it was left at room temperature for a day.

The sample was washed according to the following method: first the metal-organic gel was immersed in DMF to remove unreacted species (24 h) and washes with DMF/ethanol mixtures were then performed (every 24 h) . Finally, exchange with pure ethanol was performed (24 h).

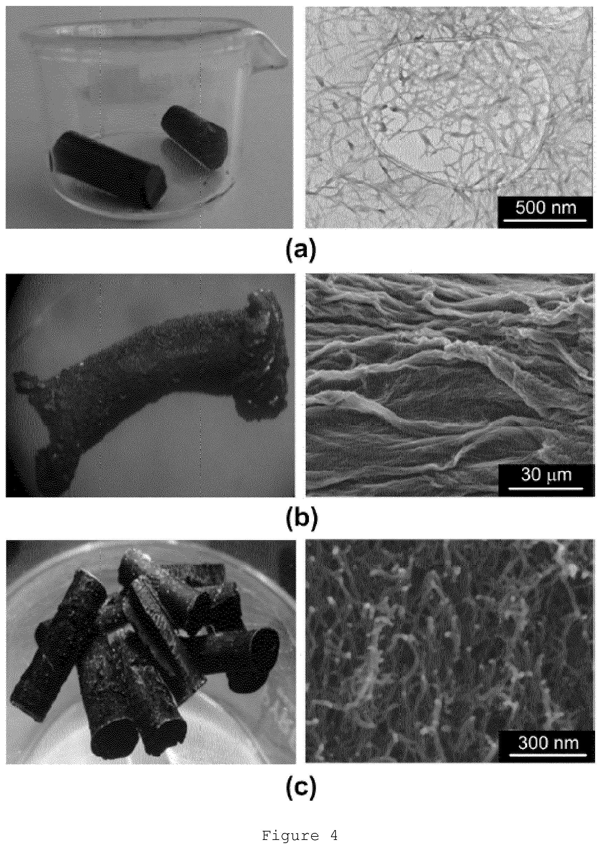

Figure 4a (left) corresponds to a photograph of the metal-organic gel in which it is observed that the metal-organic gel keeps the shape of the container where it has been synthesized. Figure 4a (right) shows an image of a fragment of the metal-organic gel obtained by transmission electron microscopy (TEM) taken in a Philips CM200 microscope.

### Example 2. Preparation of a Ni-DTO metal-organic xerogel

The corresponding xerogel was prepared by leaving the metallogel produced in Example 1 to dry at room temperature and pressure. The results are shown in Figure 4b.

### Example 3. Preparation of a Ni-DTO metal-organic aerogel

The corresponding aerogel was prepared using a Quorum Technologies® E3100 supercritical drying equipment equipped with gas inlet valves, venting valves and purge valves, and a thermal bath. The metal-organic gel produced following the method of Example 1 was first immersed in liquid CO₂ at 20°C and 50 bar for one hour. After that, ethanol was removed through the purge valve. This process was repeated five times. The sample was then dried in supercritical conditions by increasing the temperature and pressure to 38°C and 85-95 bar, respectively. Finally, the chamber was slowly vented to atmospheric pressure, keeping it at a constant temperature (38°C).

Figure 4c shows the optical image and the electron microscopy image corresponding to a Ni-DTO aerogel synthesized according to the method described in this example.

From the images shown in Figure 4, a highly porous structure consistent with the high pore volume value (3.0 cm³/g) and specific surface area value (406 m²/g) can be seen in the case of the gel and aerogel.

### Example 4. Stability tests of the metal-organic gel

Different samples of a metal-organic gel produced following the method described in Example 1 were subjected to different conditions:
1) Polydentate carboxylic acid solutions (1 M): Terephthalic acid; 2,3-pyrazinedicarboxylic acid; Trimesic acid; Isophthalic acid; Oxalic acid.
2) Amine solutions (1 M): Pentylamine; Diethanoldiamine; Pyridine; N,N,N',N'-tetramethylethylenediamine; Diethylenetriamine.
3) Conventional solvents: Dichloromethane; DMSO; Toluene; Acetonitrile; Chloroform; Diethyl ether; Pentane; Acetone.
4) Solutions at different pH (1-14) adjusted with NaOH or H₂SO₄.
5) Others: 69% HNO₃; 37% HCl; pure CH₃COOH; 25% NH₃; 30% H₂O₂; 0.2 M I₂; 2.8 M NaBH₄; 1 M KNO₃; 1 M NaCl.

Figure 2 shows how the metal-organic gel is stable in any of the conditions to which it has been subjected, without the structure thereof being altered. Particularly, the remarkable stability presented at different pH values, both acidic and basic should be highlighted.

These tests have clearly demonstrated the stability shown by the metal-organic gel of the invention at different pH values, which broadens its range of actuation as metal capturing agents. It must be added that other metal-organic gels lack stability at a low pH, which makes them unsuitable for use thereof in applications imposing extremely acidic conditions.

### Example 5. Preparation of other homometallic and bimetallic M-DTO metal-organic gels

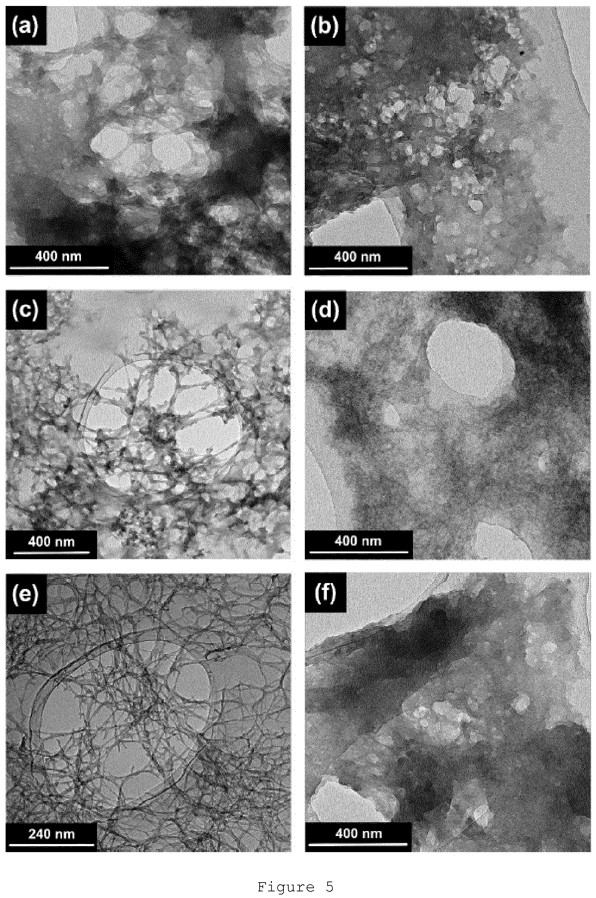

Following a method similar to that of Example 1, but changing the metal cation, M-DTO type gels where the metal centers used were Cu(II), Co(II), Fe(II), Pd(II) and Rh(II), were produced. Figure 5 shows electron microscopy images for M-DTO gels with M = Co(II), Fe(II), Pd(II), Rh(II), Ni(II)/Pd(II) and Ni(II)/Co(II). Bimetallic compounds were prepared in a similar manner using combinations of Fe/Co, Fe/Pd, Fe/Cu, Fe/Ni, Co/Ni, Co/Cu, Co/Pd, Ni/Cu, Ni/Pd and Pd/Cu. The atomic content for each metal is shown in Table 1.

**Table 1**

| | M₁ (atomic %) | M₂ (atomic %) |
|---|---|---|
| FeCoDTO | 46 | 54 |
| FePdDTO | 72 | 28 |
| FeCuDTO | 44 | 56 |
| FeNiDTO | 47 | 53 |
| CoNiDTO | 47 | 53 |
| CoCuDTO | 50 | 50 |
| CoPdDTO | 67 | 33 |
| NiCuDTO | 55 | 45 |
| NiPdDTO | 62 | 38 |
| PdCuDTO | 43 | 57 |

### Example 6. Selective metal capture tests

Different tests were conducted by immersing 1 g of metal-organic gel produced following the method of Example 1 for 24 h in aqueous solutions containing different concentrations of the following metals: Cu, Ag, Hg, Pb, Zn, Cd and Co. All the solutions were adjusted to low pH values (1-3); nevertheless, as clearly demonstrated in the preceding example, the metal-organic gel continued to remain stable even at such extreme conditions. The concentrations of each metal species were determined by ICP-AES (Inductively Coupled Plasma Atomic Emission Spectroscopy) before and after exposure to the metal-organic gel.

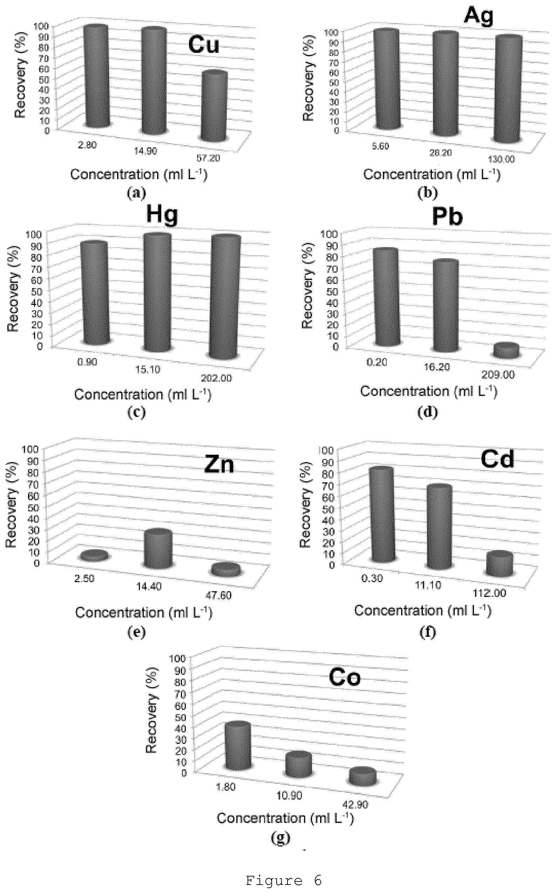

Figure 6 shows the results of the performed capture, being particularly effective in the case of Cd²⁺, Pb²⁺, Cu²⁺, Hg²⁺ and Ag⁺. The x-axis represents the initial concentration of the metal ion in the solution, whereas the y-axis shows the percentage of recovery or removal of the metal ion relative to its initial concentration. By way of example, if the percentage is 90%, it would indicate that the content of a 10 ppm metal ion solution is reduced to 1 ppm.

## Claims

1. A metal-organic gel comprising a metal-organic matrix of cross-linked nanometric fibers, **characterized in that** said nanometric fibers comprise coordination polymer chains of general formula (M-DTO)ₙ, where M is a transition metal or a mixture of at least two transition metals; DTO is dithiooxamidate; and n is the number of M-DTO repeating units forming the coordination polymer, n being a number greater than or equal to 10.

2. The metal-organic gel according to claim 1, **characterized in that** the nanometric fibers have a diameter between 2 and 300 nanometers.

3. The metal-organic gel according to claim 1 or 2, **characterized in that** the nanometric fibers have a length comprised between 0.1 and 30 µm.

4. The metal-organic gel according to any one of claims 1 to 3, **characterized in that** M is a transition metal selected from Cr, Rh, Ru, Mn, Zn, Fe, Co, Ni, Cu, Pd, Ag, Au, Cd, Pt and a mixture thereof.

5. The metal-organic gel according to any of claims 1 to 4, **characterized in that** it further comprises between 60 and 99% by weight of a solvent with respect to the total weight of the metal-organic gel.

6. A method for preparing a metal-organic gel as defined in claims 1 to 5, **characterized in that** said method comprises:
a)dissolving or dispersing a transition metal salt or a mixture thereof, in an organic solvent or a mixture of at least two organic solvents;
b)dissolving dithiooxamide and a base in an organic solvent;
c)mixing the solution or dispersion produced in step a) with the solution produced in step b); and
d)allowing the resulting mixture to stand until formation of the metal-organic gel.

7. The method according to claim 6, **characterized in that** the transition metal salt is selected from nitrate, chloride, perchlorate, bromide, sulfate, acetate and other organic carboxylates.

8. The method according to claim 6 or 7, **characterized in that** the transition metal salt is selected from a Cr, Mn, Fe, Co, Ni, Cu, Zn, Pd, Rh, Ru, Ag, Au, Cd, Pt salt, and a mixture thereof.

9. The method according to any one of claims 6 to 8, **characterized in that** the organic solvent used in step a) and the organic solvent used in step b) are independently selected from N,N-dimethylformamide (DMF); N,N-diethylformamide (DEF); dimethylsulfoxide (DMSO); N,N-dimethylacetamide (DMA); alcohols such as methanol, ethanol, iso- or n-propanol, butanol; tetrahydrofuran and a mixture thereof.

10. The method according to any one of claims 6 to 9, **characterized in that** the base used in step b) is selected from sodium hydroxide, potassium hydroxide, sodium methoxide, ammonia and alkylamine.

11. A metal-organic gel obtainable by the method defined in any of claims 6 to 10.

12. The method according to any of claims 6 to 10, **characterized in that** it further comprises at least one washing step for washing the metal-organic gel.

13. The method according to claim 12, **characterized in that** at least one washing step is performed in the presence of an alcohol.

14. The method according to any one of claims 6 to 10 and 12 to 13, **characterized in that** it further comprises a drying step for drying the metal-organic gel at room temperature and pressure conditions or by means of providing external heat and/or by applying a vacuum.

15. A metal-organic xerogel obtainable by the method defined in claim 14.

16. The method according to any one of claims 6 to 10 and 12 to 13, **characterized in that** it further comprises a supercritical drying step for drying the metal-organic gel.

17. The method according to claim 16, **characterized in that** the supercritical drying is performed by first immersing the metal-organic gel in liquid CO₂, and then imposing supercritical conditions for drying at a temperature greater than 31°C and a pressure between 60 and 150 bar.

18. A metal-organic aerogel obtainable by the method defined in any of claims 16 to 17.

19. The metal-organic aerogel according to claim 18, **characterized in that** it has a specific surface area between 100 and 800 m²/g.

20. The metal-organic aerogel according to claim 18 or 19, **characterized in that** it has pore volumes comprised between 0.5 and 10 cm³/g.

21. The metal-organic aerogel according to any one of claims 18 to 20, **characterized in that** it has an average pore size between 2 and 300 nm.

22. Use of a gel as defined in any one of claims 1 to 5 and 11, or of a metal-organic aerogel as defined in any of claims 18 to 21, in chemical species capture, separation and/or catalytic processes, contaminated water cleanup or metal recovery processes, and in analytical applications such as passive sampling.
